# EUROPEAN PATENT SPECIFICATION

(11) **EP 3 889 576 B1**
(45) Date of publication and mention of the grant of the patent: **25.03.2026**
(21) Application number: 18941524.3
(22) Date of filing: 01.12.2018
(51) Int. Cl.: G01N 15/14, G01N 33/577, C12N 5/078, G01N 1/30, G01N 1/38, G01N 1/40, G01N 33/50, G01N 15/1429, G01N 15/10, G01N 15/00, G01N 15/01

(54) **FLOW CYTOMETRY TESTING METHOD FOR LYMPHOCYTE IN IMMUNE CELL**
DURCHFLUSSZYTOMETRIEPRÜFVERFAHREN FÜR LYMPHOZYTEN IN EINER IMMUNZELLE
PROCÉDÉ DE TEST DE CYTOMÉTRIE EN FLUX POUR UN LYMPHOCYTE DANS UNE CELLULE IMMUNITAIRE

(43) Date of publication of application: 06.10.2021
(73) Proprietor: Mingdao Innovation (Beijing) Medical-Tech Co., Ltd., Beijing 101111 (CN)
(72) Inventor: YAO, Weili, Beijing 101111 (CN)
(74) Representative: Günther, Constantin
(86) International application number: PCT/CN2018/118802
(87) International publication number: WO 2020/107496

(56) References cited:
- CN-A- 103 760 349
- CN-A- 104 133 058
- CN-A- 104 941 001
- CN-A- 105 547 971
- CN-A- 107 063 982
- CN-A- 107 063 982
- US-A- 5 087 295
- US-A1- 2003 022 249
- US-A1- 2003 104 461
- US-A1- 2017 343 545
- US-A1- 2018 246 105
- Instructions for Use CytoFLEX Beckman Coulter https://pedsresearch.org/uploads/blog/doc/Cytoflex-Manual.pdf XP093322243
- KOHRT, H.E. ET AL.: "Targeting CD137 Enhances the Efficacy of Cetuximab", THE JOURNAL OF CLINICAL INVESTIGATION, vol. 124, no. 6, 30 June 2014 (2014-06-30), XP055218510, DOI: 20190814190722A

## Description

### Technical Field

The present application relates to the field of medicines, and in particular to a flow cytometric detection method for lymphocyte in immune cells.

### Background

The human immune system is usually divided into: immune organs and immune tissues, immune cells (phagocytes and lymphocytes), immune active substances (antibodies, lysozyme, complement, immunoglobulin, interferon, interleukin, tumor necrosis factor and other cytokines). Among them, the traditional detection of immune lymphocytes usually includes adding fluorescent antibody to 100 µl peripheral blood, incubating for 15-30 minutes, then lysing red blood cells with hemolysin, washing, adding machine buffer solution, and testing with flow cytometer.

CN201680006060.1 discloses a method for examining an immune state, which is performed based on the ratio of each subgroup of immune cells: T, B, NK, NKT and T cells (subdivided into CD4+T cells and CD8+T cells). This invention has the following technical shortcomings: 1. there is a waste of antibody and reagent materials; 2. there is no definition of DC cell subtype having innate immunity; 3. the definition of individual lymphocyte subpopulations is not comprehensive, and the function of lymphatic immune system is not considered systematically, so that the division of subpopulations is not comprehensive and balanced; 4. lysing red blood cells by using red blood cell lysis solution after dyeing immunofluorescent antibody results in the unclean background and too much debris, which intends to cause data errors; 5. for a small number of lymphocyte subpopulations, the method cannot detect or the results are not accurate; and 6. this method is prone to over-staining or under-staining, resulting in inaccurate data.

CN201410486089.7 discloses a method for lymphocyte immunophenotyping and a kit thereof, which further divides T cells into the same types of T(subdivided into CD4+T cells and CD8+T cells) , B, NK, NKT, based on which, several T cells are defined by the combination of CD45RA and CD27, and four types of B cells are defined by CD19, CD24, CD38, and CD27. This invention has the following technical shortcomings: 1. there is a waste of antibody and reagent materials; 2. there is no definition of DC cell subtype having innate immunity; 3. the definition of individual lymphocyte subpopulations is not complete, and the function of lymphatic immune system is not considered systematically, so that the division of subpopulations is not comprehensive and balanced; 4. lysing red blood cells by using red blood cell lysis solution after dyeing immunofluorescent antibody results in the unclean background and too much debris, tending to cause data errors; 5. for a small number of lymphocyte subpopulations, the method cannot detect or the results are not accurate; and 6. this method is prone to over-staining or under-staining, resulting in inaccurate data.

Therefore, a more comprehensive and balanced method for detecting lymphocyte subpopulations is still a research hotspot, and there is still no relevant report at present. CN107063982A and US2018/246105 A1 disclose relevant methods for detecting immune cells using lymphocyte separation solutions.

### SUMMARY

In view of the above technical status, the present application provides a method for detecting lymphocytes in immune cells according to claim 1.

In the method of the present application, as one of the embodiments, the pre-cooling temperature in step a) is a temperature commonly used for flow cytometric detection in the art, including but not limited to 2-8°C in the present application.

In the method of the present invention, the PBS-EDTA solution in step a) or step b) is a mixed solution containing 0.005-0.05M PBS and a final concentration of 2-3mM EDTA with pH of 7.2-7.4;
In the present application, as one of the embodiments, the 0.005-0.05M PBS, for example, can be 0.005M PBS, 0.01M PBS, 0.02M PBS, 0.03M PBS, 0.04M PBS or 0.05M PBS.

In the method of the present invention, the concentration of cells in the lymphocyte sample is 1.5 X 10⁶-5 X 10⁶/ml, further preferably 1.5-3.0 X 10⁶/ml.

In the method of the present application, as one of the embodiments, the step a) further includes: the volume of the lymphocyte sample is a volume suitable for detection, including but not limited to 50-200 µl in the present application.

In the method of the present application, as one of the embodiments, in step b), the flow speed is adjusted to a medium speed range of 30-44µl/min; as one of the further embodiments, the flow speed is adjusted to a medium speed range of 35-40µl/min; as an example, the flow speed can be adjusted to 35µl/min, 36µl/min, 37µl/min, 38µl/min, 39µl/min or 40µl/min.

In the method of the present application, as one of the embodiments, a preheating time in step b) can be the time suitable for detection, which can be adjusted by those skilled in the art, and it is preferably 5-10 minutes in the present application.

In the method of the present application, as one of the embodiments, the amount of PBS-EDTA solution added into the sample tube in step b) can be determined by those skilled in the art according to the detection equipment and conditions, for example, including but not limited to 3ml.

In the method of the present invention, step c) further includes setting conditions as follows: 3000-20000 cells in the gate, preferably 15000 cells in the gate, and collecting samples.

In the method of the present invention, the flow cytometric cell sample is prepared as described in claim 1.

In the method of the present invention, the PBS solution is 0.005-0.05M PBS solution at pH 7.2-7.4; As one of the embodiments, the PBS buffer solution can contain calf serum and/or EDTA, and the concentration of the calf serum or EDTA can be the concentration commonly used in literature.

In the method of the present invention, the PBS-EDTA solution is a mixed solution at pH 7.2-7.4 containing 0.005-0.05M PBS and a final concentration of 2-3mM EDTA.

In the method of the present application, as one of the embodiments, step 1) further includes centrifuging conditions of 1500-3500rpm for 5-30min, preferably 1500-2900rpm for 15-20min.

In the method of the present application, as one of the embodiments, step 2) further includes diluting blood cell precipitate with PBS solution by a volume ratio of 1:0.5 to 1:2, preferably 1:1.

In the method of the present application, as one of the embodiments, the lymphocyte separation solution in step 3) can be prepared by those skilled in the art according to the existing technology, or can be commercially purchased. The lymphocyte separation solution from GE company, an American reagent company, is preferred in the present application.

In the method of the present application, as one of the embodiments, step 3) further includes centrifuging conditions of 1500-3500rpm, 10-20min and 4°C; preferably, 1500-2900rpm, 15-20min and 4°C; in the method of the present application, as one of the embodiments, in step 4), if necessary, an appropriate amount of suspension can be selected before centrifuging and counted with a hematology analyzer for subsequent adjustment of cells concentration.

In the method of the present application, as one of the embodiments, step 4) further includes centrifuging conditions of 1500-3500rpm, 5-20min and 4°C; as one of the further embodiments, the centrifuging conditions are 1500-2900rpm, 5-10min and 4°C.

In the method of the present application, as one of the embodiments, PBS solution is added to the centrifuge tube in step 4) until the volume is 10ml-15ml.

In the method of the present application, as one of the embodiments, the amount of diluted lymphocytes obtained in step 4) and added to the flow tube in step 5) can be an amount commonly used in the art, as an example, including but not limited to 100 µl;

In the method of the present application, as one of the embodiments, as an example, the incubation time in step 5) can include, but not limited to, 10-30 minutes.

In the method of the present application, as one of the embodiments, step 6) further includes centrifuging conditions of 1500-2900rpm for 5-10min.

In the method of the present application, as one of the embodiments, the amount of PBS solution added in step 6) is 2ml.

In the method of the invention, as one of the embodiments, the PBS-EDTA solution in step 7) is an EDTA mixed solution at pH 7.2-7.4 containing 0.005-0.05M PBS and a final concentration of 2-3mM EDTA; the PBS solution is 0.005-0.05M PBS solution at pH 7.2-7.4; alternatively, the PBS buffer solution can contain calf serum and/or EDTA, and the concentration of calf serum or EDTA can be the concentration commonly used in the art.

In the method of the present application, as one of the embodiments, the centrifuging conditions in step 7) are 1500-2900rpm for 5-10min. An alternative way of preparing the lymphocyte sample for flow cytometry detection according to step a) of claim 1 is provided in independent method claim 14.

By using the method of the present application, the lymphocyte sample can be easily obtained and quantified, and the detection of all detection indexes can be realized only by a small amount of in vitro peripheral blood samples; at the same time, it can realize the detection of cells including but not limited to DC cells (dendritic cells) and the like.

The specific experimental steps of the detection method of the present application are divided into four steps: acquisition of in vitro blood sample, cell separation, antibody incubation and flow cytometric detection. Based on the principle of antigen-antibody binding, the present application adopts antibodies combined with different fluorescence to bind specific antigens on the surface of cells, so as to label such subpopulations.

The proportion and number of such cells can be analyzed during flow cytometric detection.

Acquisition of in vitro blood samples: obtained by collecting peripheral blood from blood collection vessels in vitro.

Cell separation: the blood sample separation method of the present application can save more material cost and time cost, and provide faster and more accurate experimental results.

In a verified experimental method, the peripheral blood is separated and washed with lymphocyte separation solution, diluted to the specified range of concentration, and stored at 4°C for use.

Antibody incubation: fluorescent antibody corresponding to specific antigen on the surface of each type of cells were selected; and the isolated cells were diluted to an appropriate concentration, incubated according to the verified concentration of fluorescent antibody and verified time, then washed with washing solution to remove excessive antibody, and mixed with flow cytometric buffer solution.

Flow cytometric detection: a flow cytometer is used for detection and analysis.

The experimental method of the present application is different from the traditional flow cytometric method. The present application highly enriches lymphocyte, and the cells that affect the detection results such as red blood cells and granulocytes are removed., so that many cell subpopulations with low expression or trace amount of expression can be clearly presented, providing obvious populations and facilitating analysis and research. From another point of view, by using high purity lymphocyte separated by the present application, the efficiency of binding antibody per unit volume will be much higher, and more than half of the antibody cost and antibody incubation time can be saved.

Compared with the prior art, the present application has the following advantages: 1. The lymphocyte is separated and enriched by using the lymphocyte separation solution, which ensures reliable and accurate detection results and save antibodies and reagents; 2. By detecting enriched lymphocyte subpopulations, it can effectively analyze lymphocyte subpopulations with small number of cells and ensure effectively and reasonably staining the lymphocyte subpopulations as defined; 3 The lymphocyte subpopulations that can be detected by the method of the present application cover an immune recognition early-warning system, cellular immune system, humoral immune system and non-specific immune cell system, so the detection results can comprehensively reflect the state of the lymphocyte system; 4. The present application can clearly define two subtypes of DC cells; 5. The invention can comprehensively analyze and detect the lymphocyte subpopulations, providing a scientific basis for comprehensive understanding of the immune status.

### Brief Description of Drawings

Fig. 1: detection diagram of lymphocyte subsets in Example 2;
Fig. 2: detection diagram of lymphocyte subsets in Example 2;
Fig. 3: detection diagram of lymphocyte subsets in Example 3;
Fig. 4: detection diagram of different lymphocyte subsets in example 3;
Fig. 5: detection diagram of DC cell subtypes in Example 3;
Fig. 6: comparison of cell populations of the sample obtained by the method of Example 1 and the sample obtained by the lysis method in the literature in Example 4; (the upper three figures show the flow cytometric diagrams obtained by the lysis method, and the lower three figures show the flow cytometric diagrams obtained by separation of corresponding samples in the upper three figures.)
Fig. 7: comparison diagram of cell enrichment between the sample obtained by the method of Example 1 and the sample obtained by the lysis method in the literature in Example 4;
Figure 8: comparison diagram of enrichment of different types of cells between the sample obtained by the method of Example 1 and the sample obtained by the lysis method of literature in Example 4.

### Detailed description

The following embodiments and test examples are used to further elaborate the application, not intended to limit the effective scope of the present application in any way.

### Example 1 Preparation of PBMC samples needed for flow cytometry

### 1. Purpose

preparing PBMC samples needed for flow cytometric detection (also referred to as "samples" in the context of the present application)

### 2. Principle

There are relatively complete antigens or receptors remained on the surface of living cells. Fluorescent labeled antibodies are used to bind to corresponding antigens on the surface of cells, and fluorescence intensity and positive percentage are measured to provide the density and distribution of corresponding antigens.

### 3. Preparation of instruments and consumable materials

### 3.1 Instruments

Super Clear Workbench, Era Beili low speed centrifuge DT5-4, a vortex mixer, pipettes of various ranges, and a blood cell counter

### 3.2 Consumable materials

15ml centrifuge tube, 10ml pipette, 1.5ml centrifuge tube, a flow tube, 3ml pasteurizer pipette, and pipette tips of various ranges.

### 3.3 Formulations of reagents

| Name | Stock solution | Formulation |
|---|---|---|
| 1×PBS | 10×PBS | stock solution: purified water =1:9 |
| 0.1% formalin | 10% formalin | stock solution:1×PBS = 1:9 |
| PBS solution | 1×PBS | 1×PBS (0.01M PBS , pH=7.2-7.4) |
| 1×hemolysin | 10×hemolysin | stock solution: purified water = 1:9 |
| PBS-EDTA solution | 1×PBS | adding 0.5M EDTA to 1×PBS (0.01M PBS; pH=7.2-7.4) until the final concentration of EDTA is 2.5mM |

### Routine flow cytometric antibodies;

### 4. Operation procedure (illustrated by taking 5ml in vitro original blood sample as an example)

### 4.1. Separation of in vitro peripheral blood sample (PBMC)

(1) obtaining 0.2ml in vitro original blood sample and counting;
(2) adding 5ml blood to a 15ml centrifuge tube, centrifuging at 1500-2900rpm for 15-20min, and discarding upper plasma;
(3) diluting cell precipitate of the rest of the original blood with PBS solution by volume;
(4) providing a centrifuge tube and adding lymphocyte separation solution having a volume equal to the volume of the blood cell diluent to the centrifuge tube by using a pipette;
(5) adding blood cell diluent into the centrifuge tube in step (4) slowly for keeping the layer of the separation solution clear;
(6) slowly placing the centrifuge tube into a centrifuge, balancing, and centrifuging at 1500-2900rpm for 15-20min;
(7) slowly suctioning and discarding the supernatant with a pipette after centrifuging;
(8) pipetting middle buffy coat cells into a new centrifuge tube, adding PBS solution to a volume of 10ml, counting 200µl of the cell suspension on a blood cell counter, and centrifuging the rest of the cell suspension at 1500-2900rpm for 5-10min;
(9) discarding the supernatant after centrifuging, and removing residual liquid by using a pipette. According to the result of counting, appropriate amount of pre-cooled PBS solution at 4°C was added, so as to adjust the density of cells to 1.5-3.0 X 10⁶/ml, for example, 2X 10⁶/ml.

### 4.2 Preparation of flow cytometric samples (the temperature was controlled in this operation, and the samples and antibodies were operated in an ice box throughout the operation)

### (1) Sample staining

A flow tube was added with 100µl lymphocyte suspension, added with flow cytometric antibody, gently shook in a vortex mixer to fully mix the antibody with the cells, and incubated in the dark at 2-8°C for 20 minutes.

### (3) Sample washing

After incubating, the supernatant was discarded, and the cells were resuspended in 2ml pre-cooled PBS solution, and centrifuged at 1500-2900 rpm for 5-10 minutes.

### (4) Completion of samples

① If the detection on a flow cytometer is performed immediately , the supernatant is discarded and the cells are mixed in 400µl pre-cooled PBS-EDTA solution for flow cytometric detection;
② If the detection cannot be performed immediately, cells are suspended in 500µl 0.05-5% formalin, vortex mixed, and left to stand at 4°C in the dark. Before detection, each of the tubes is added with 2ml PBS solution, and centrifuged at 1500-2900 rpm for 5-10 minutes to discard the supernatant, and the cells are mixed with 400µl pre-cooled PBS-EDTA solution for detection on a flow cytometer.

### 5. Notes

5.1 the antibodies and cells should be kept at low temperature (4°C) during the operation.

5.2 after adding the antibodies, the antibodies and the cells should be fully mixed.

### Example 2 Detection of PBMC samples

### 2.1 instruments, reagents and samples:

Sample: prepared by the method of Example 1 from in vitro blood samples;
Flow cytometer: ACEA NovoCyte

### 2.2 detection method:

a) checking the status of the cytometer before starting up;
b) warming up the flow cytometer system for 5-10 minutes and performing perfusion (for about 15 minutes);
c) performing quality control procedure and proceeding to the next step after qualification;
d) setting the parameters of the flow cytometer, adjusting the voltage of each of detection channels, and setting the liquid flow speed to 35-40 µL/min;
e) vortex vibrating the prepared flow cytometric samples for 3 seconds, and subjecting the samples to detection under the conditions of: 15000 cells in the gate; and collecting samples.

### 2.3 results of detection

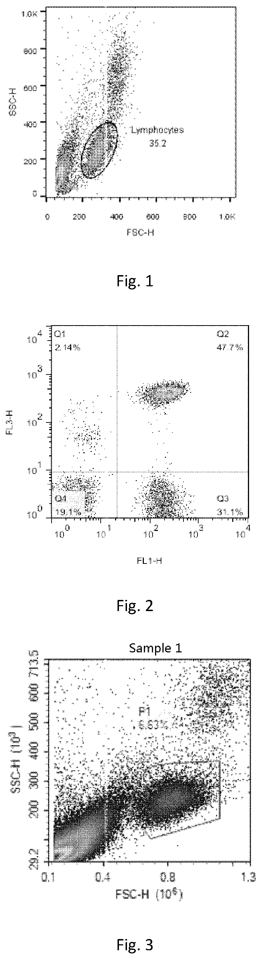

It can be seen from Fig. 1 that, in the plot of FSC vs SSC, gate 1: lymphocyte population, lymphocyte subpopulations can be clearly identified, and the data will be more reliable and accurate since it is populations of cells that are main analyzed.

It can be seen from Fig. 2 that: (1) gate 1 is selected to display FL1 and FL3 area in gate 1; (2) select cross gate 2 in the gate 1: CD3+CD4+ cell population (Q2 region) is selected. The lymphocyte subpopulations to be analyzed are well separated, the gate is more easier to set, and the data is more accurate.

### Example 3 Detection of PBMC samples

### 3.1 Instruments, reagents and samples:

Sample: prepared by the method of Example 1 from 5ml in vitro blood samples;
Flow cytometer: ACEA NovoCyte

### 3.2 Detection method:

a) checking the status of the cytometer before starting up;
b) warming up the flow cytometer system for 5-10 minutes and performing perfusion (for about 15 minutes);
c) performing quality control procedure and proceeding to the next step after qualification;
d) setting the parameters of the flow cytometer, adjusting the voltage of each of detection channels, and setting the liquid flow speed to 35-40 µL/min;
e) vortex vibrating the prepared flow cytometric samples, and subjecting the samples to detection under the conditions of: 15000 cells in the gate; and collecting samples.

### 3.3 Results of detection

1) Fig. 3 shows the analysis of lymphocyte subpopulations obtained from the peripheral blood of one healthy person by lymphocyte separation method, in which a population of lymphocyte is in circled gate P1 . As can be seen from Fig. 3, the background is clean, and the population of lymphocyte and population of cell debris are well and ideally separated.

It can be seen that, the present application separates and enriches lymphocytes by using lymphocyte separation solution, which are obviously divided, ensuring reliable and accurate detection results while reducing antibodies and reagents.

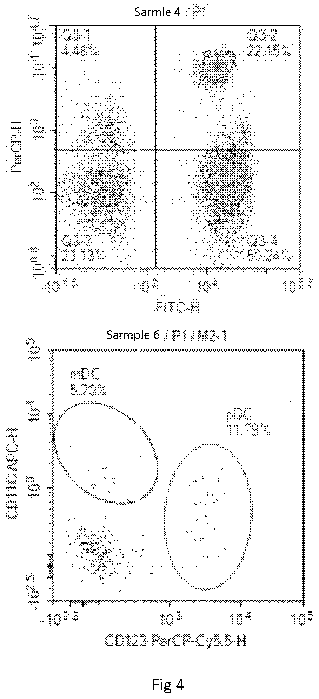

2) Fig. 4 shows that, through the detection of enriched lymphocyte subpopulations, lymphocyte subpopulations with relatively small number of cells are effectively analyzed, and aslo the specific lymphocyte subpopulations can be effectively stained; In Fig. 4, for sample 4 on the left, Q3-2 is CD8+T cells, which are obviously separated via separation and enrichment so that the subpopulations can be well analyzed. The right figure of Fig. 4 shows the cell subpopulations with a small proportion of lymphocyte subpopulations, that is, DC cell subpopulation, which can also be accurately analyzed after enrichment.

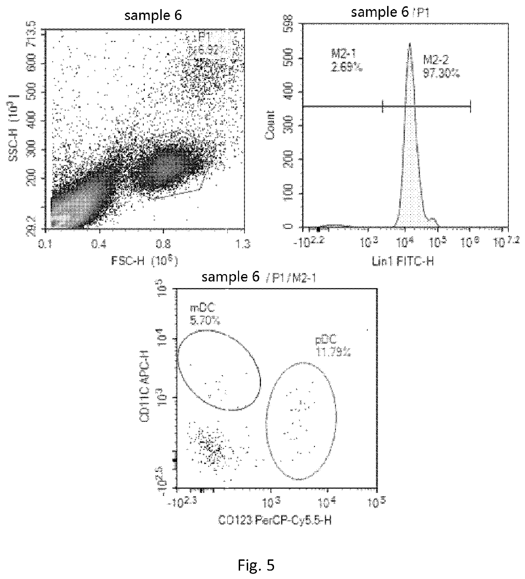

3) Fig. 5 clearly defines two subtypes of DC cells, which otherwise should not be identified in conventional lymphocyte subpopulation analysis. The two subtypes of cells were enriched by separation. As shown in the right 1 of Figure 5, the population is obviously separated and the boundary is clear.

### Example 4 Comparison between the sample prepared in the method of the present application and the sample obtained by the hemolysin lysis method

### 4.1 samples and reagents:

The lymphocyte sample of the present application: prepared by the method of Example 1 from in vitro peripheral blood sample;
Comparative lymphocyte samples: prepared by the method according to following section 4.2.1;
5ml anticoagulant blood sample;
1 X PBS: prepared from 10 X PBS solution: pure water = 1:9 by volume;
PBS solution: 1 X PBS;
PBS-EDTA solution: prepared by adding 0.5m EDTA to 1 X PBS until the final concentration of EDTA is 2.5 mM;
Conventional flow cytometric antibodies: anti-CD3 antibody, lin1[Lineage cocktail 1], anti-CD123 antibody, anti-CD11 antibody, CD3 FITC, CD8PerCP, CD4PerCP.

### 4.2 Testing method:

### 4.2.1 hemolysin lysis method:

(1) Sample staining: adding 100µl anticoagulant blood sample into each of flow tubes, and adding flow cytometric antibody to label lymphocyte subpopulations respectively, so that the antibody was fully mixed with cells, and incubating the flow tubes in the dark at 2-8°C for 20 minutes.
(2) Lysis: adding 2 ml 1 × BD hemolysin (i.e. red blood cell lysis solution) to the flow tube, and performing lysis for 5 minutes.
(3) Sample washing: 5 minutes later, centrifuging at 1500-2900 rpm for 10 minutes, discarding the supernatant, adding 2ml pre-cooled PBS solution to resuspend the cells, centrifuging at 1500-2000 rpm for 5-10 minutes, discarding the supernatant, adding 400µL PBS-EDTA solution at 2-8°C, and evenly mixing.

### 4.2.2 method for detection on a flow cytometer

a) before starting up, checking the volume of sheath barrel, waste liquid barrel, detergent and flushing agent, adding enough sheath liquid, and pouring out waste liquid;
b) warmed up the flow cytometric system for 5-10 minutes and perform perfusion (for about 15 minutes);
c) performing quality control procedure and proceeding to the next step after qualification;
d) setting the parameters of the flow cytometer, adjusting the voltage of each of detection channels, and setting the liquid flow speed to 35-40 µL/min;
e) vortex vibrating the prepared flow cytometric samples of the present application and comparative lymphocyte samples for 3 seconds, respectively, and subjecting the samples to detection under the conditions of: 15000 cells in the gate; and collecting samples.

### 4.3 results of experiments:

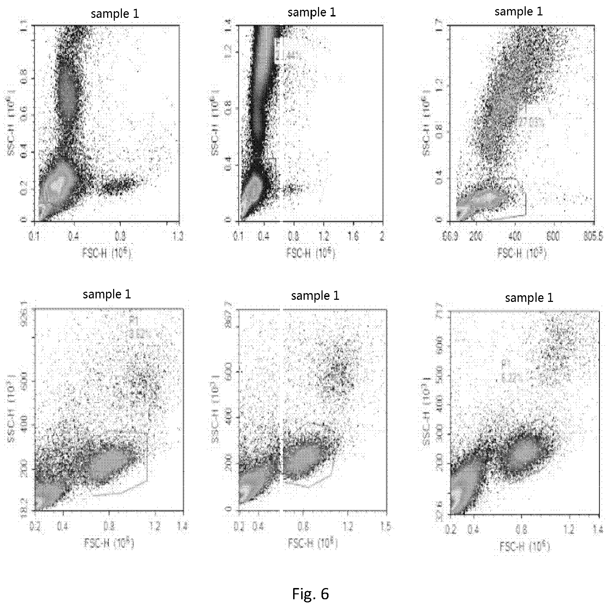

### 4.3.1 through comparison, it can be seen that there is much debris in the sample obtained by lysis, and the background is not clean, which influences the collection and analysis of flow cytometry data, and the compensation adjustment of lymphocytes may cause bias phenomenon; and the present method (method by using lymphocyte separation solution) provides clean background and clear and obvious populations (see Fig. 6).

In particular, for the lymphocytes in the gate circled in the lymphocyte subpopulation analysis obtained by lysis method, it can be seen from the figure that the lymphocyte subpopulation and the cell debris population is not ideally separated (see the three figures in the first row of Fig. 6).

For the lymphocytes in the gate circled in the lymphocyte subpopulation analysis obtained by the present method, it can be seen for the figure that the lymphocyte subpopulations and cell debris population are well and ideally separated (see the third figures in the second row of Fig. 6).

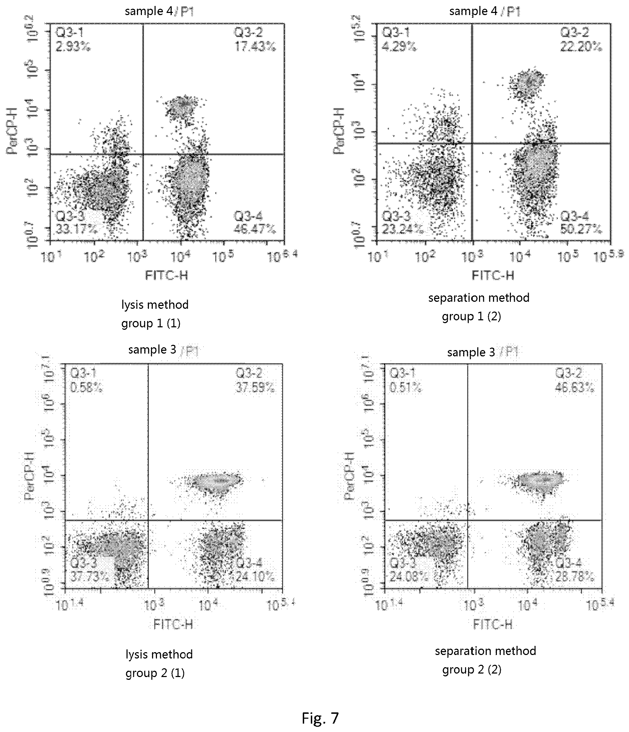

4.3.2 It can be seen from Fig. 7 that, in the present method, when the fluorescent antibody is used, since the number of lymphocytes is counted and the fluorescent antibody is added in proportion, more than 70% of the antibody is saved. The amount of combined antibody used in the present application is 15µL, which is 60µL in the lysis method. The method of the present application does not cause excessive or insufficient antibody. It can be seen from Fig. 7 that CD3 FITC and CD8PerCP were labeled in group 1, and CD3 FITC and CD4PerCP were labeled in group 2. Due to the different samples obtained by different treatment methods, for the same separated peripheral blood sample, the result showed that, the proportion of double positive groups in the sample obtained by the lysis method is significantly reduced, and the amount of antibody cannot be quantified; while for the sample obtained by the method of the present application, the antibody can be quantitatively added according to the amount of cells. In addition, it is obvious that the cells obtained by the separation method are significantly enriched.

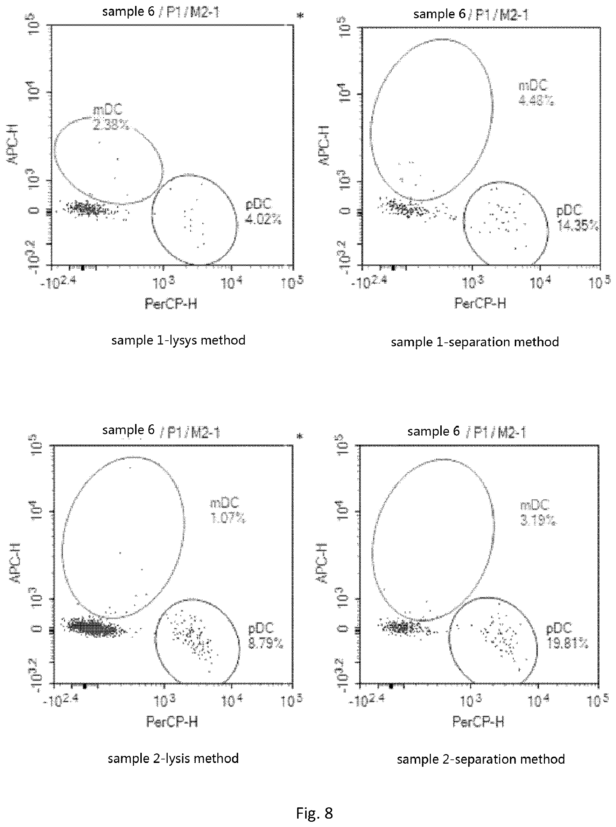

4.3.3 it can be seen from Fig. 8 that the sample prepared by the present application has enrichment effect for the sample with small proportion of cell subpopulations. For the same separated peripheral blood sample, for the MDC and PDC accounting for small proportion, the observation results of the samples as obtained are different in the lysis method and the method of the present application, and the cell subpopulations in the samples obtained by the present application are enriched, which can be shown from the number and percentage of cells.

## Claims

1. A method for detecting lymphocytes in immune cells, comprising the steps of:
a) adding lymphocyte samples stained with immunofluorescence antibody into a pre-cooled PBS-EDTA solution, and mixing and preparing the cells for flow cytometry detection;
b) starting up and preheating a flow cytometer system, adjusting the flow speed to a low-speed range of 10-29µl/min or a medium speed range of 30-44µl/min, then adding PBS-EDTA solution into a sample tube, in order to flush a nozzle system of liquid stream;
c) adding the sample of homogenous cells obtained in step a) to the sample tube and testing thereof with setting conditions of 3000-20000 cells in a gate during testing and collecting samples;
the PBS-EDTA solution in step a) or step b) is
a mixed solution containing 0.005-0.05M PBS and a final concentration of 2-3mM EDTA with a pH of 7.2-7.4;
Whereby the lymphocyte samples for flow cytometry detection to be used in step a) are prepared by the following method:
1) centrifugation of *in vitro* anticoagulant blood samples to separate plasma and blood cell precipitation to obtain blood cell precipitation;
2) diluting the blood cell precipitation with PBS solution to obtain blood cell diluent;
3) adding slowly the blood cell diluent to a centrifuge tube containing an equal volume of lymphocyte separation solution, and then centrifugation thereof to remove the plasma to obtain the middle buffy coat cells diluent;
4) adding the buffy coat cells obtained in step 3) into a centrifuge tube, adding PBS solution under stirring to obtain a suspension, centrifuging, removing supernatant, and then adding PBS solution to adjust the cell concentration to 1.5 × 10⁶/ml - 5 ×10⁶/ml;
5) adding the lymphocyte cells obtained in step 4) after adjusting the concentration thereof into a flow tube; and adding flow cytometric antibody to label lymphocyte subpopulations, evenly mixing, standing at 2-8°C and incubating without light; whereby the antibodies comprise anti-CD3 antibody, lin1[Lineage cocktail 1], anti-CD123 antibody, anti-CD11 antibody, CD3 FITC, CD8PerCP, and CD4PerCP;
6) adding PBS solution, mixing, and centrifuging to remove unbound antibody;
7) completion of samples; whereby
i) If a detection on a flow cytometer is performed immediately, the supernatant is discarded and the cells are evenly mixed with PBS-EDTA solution pre-cooled to 2-8°C for flow cytometric detection;
ii) If the detection cannot be performed immediately, cells are directly resuspended by adding 0.05-5% formalin, mixed, and left to stand at 2-8°Cin the dark; and before detection, each of the tubes is added with PBS solution, centrifuged to discard the supernatant, and added with PBS-EDTA solution at 2-8°C to evenly mix cells for flow cytometric detection;
and the PBS solution is 0.005-0.05M PBS solution at pH 7.2-7.4.

2. The method according to claim 1, **characterized in that**, step a) further includes: the volume of the lymphocyte sample is 50-200 µl.

3. The method according to claim 1, **characterized in that**, in step b), the flow speed is adjusted to a medium speed range of 35-40µl/min.

4. The method according to claim 1, **characterized in that**, step b) further includes a preheating time of 5-10 minutes.

5. The method according to claim 1, **characterized in that**, step c) further includes setting conditions of 15000 cells in the gate during testing, and collecting samples.

6. The method according to claim 1, **characterized in that**, step 1) further comprises centrifuging conditions of 1500-3500rpm for 5-30min, preferably 1500-2900rpm for 15-20min.

7. The method according to claim 1, **characterized in that**, step 2) further comprises diluting blood cell precipitate with PBS solution by a volume ratio of 1:0.5 to 1:2, preferably 1:1.

8. The method according to claim 1, **characterized in that**, step 3) further comprises centrifuging conditions of 1500-3500rpm, 10-20min and 4°C; preferably, 1500-2900rpm, 15-20min and 4°C.

9. The method according to claim 1, **characterized in that**, step 4) further comprises centrifuging conditions of 1500-3500rpm, 5-20min and 4°C; preferably centrifuging conditions of 1500-2900rpm, 5-10min and 4°C.

10. The method according to claim 1, **characterized in that**, PBS solution is added to the centrifuge tube in step 4) until the volume is 10ml-15ml.

11. The method according to claim 1, **characterized in that**, step 6) further comprises centrifuging conditions of 1500-2900rpm for 5-10min.

12. The method according to claim 1, **characterized in that**, the amount of PBS solution added in step 6) is 2ml.

13. The method according to claim 1, **characterized in that**, the centrifuging conditions in step 7) are 1500-2900rpm for 5-10min.

14. A method for detecting lymphocytes in immune cells comprising the steps of:
a) adding lymphocyte samples stained with immunofluorescence antibody into a pre-cooled PBS-EDTA solution, and mixing and preparing the cells for flow cytometry detection;
b) starting up and preheating a flow cytometer system, adjusting the flow speed to a low-speed range of 10-29 µl/min or a medium speed range of 30-44µl/min, then adding PBS-EDTA solution into a sample tube, in order to flush a nozzle system of liquid stream;
c) adding the sample of homogenous cells obtained in step a) to the sample tube and testing thereof with setting conditions of 3000-20000 cells in a gate during testing and collecting samples;
the PBS-EDTA solution in step a) or step b) is a mixed solution containing 0.005-0.05M PBS and a final concentration of 2-3mM EDTA with a pH of 7.2-7.4;
whereby the lymphocyte samples for flow cytometry detection to
be used in step a) are prepared by the following method:
(I) Isolation of Human Peripheral Blood Mononuclear Cells from Peripheral Blood;
(1) taking 0.2 mL of the isolated original blood sample for counting, and retaining 1 mL of the isolated original blood sample with labeled information and stored at 4°C;
(2) centrifuging the blood in a centrifuge tube at 1500-2900 rpm for 15-20 minutes, and storing the upper plasma in a 2ml cryopreservation tube at - 80°C;
(3) diluting the cell precipitate of the rest of the blood with PBS solution by a volume ratio of 1:1;
(4) prepare a 15ml centrifuge tube and fill the centrifuge tube with lymphocyte separation solution of equal volume to the blood cell diluent;
(5) slowly add the blood cell diluent to the centrifuge tube in step (4), keeping the separation fluid well stratified;
(6) placing the centrifuge tube into a low-speed centrifuge, balancing, and centrifuging at 1500-2900 rpm for 15-20 mins;
(7) after centrifugation, the upper plasma is slowly absorbed with a 10ml pipette or a 3ml Pasteur pipette and discarded;
(8) pipetting middle buffy coat cells by circles into a new 15ml centrifuge tube, adding PBS solution to a volume of 10 ml, counting 200µl of cell suspension on a whole blood cell counter for subsequent adjustment of cell concentration, and centrifuging the rest of the cell suspension at 1500-2900 rpm for 5-10 mins; and
(9) centrifuging, discarding the supernatant, and, according to the result of counting, adding appropriate amount of pre-cooled PBS solution at 2-8°C to adjust the cell density to 1.5-2.0 × 10⁶/ml;
(II) Preparation of flow cytometric samples
(10) sample staining
Each of the flow tubes is added with 100µl cell suspension, added with corresponding flow cytometric antibody whereby the antibody is evenly mixed with cells, and incubating in the dark at 4°C for 10-30 minutes, preferably 15-20 minutes and most preferably 20 minutes.
(11) sample washing and solution adding After incubating, resuspending the cells in 2ml PBS solution at 2-8°C, centrifuging at 1500-2900 rpm for 5-10 min and discarding supernatant after centrifuging; whereby
i) If a detection on a flow cytometer is performed immediately, the cells are evenly mixed with 400µl pre-cooled PBS-EDTA solution ready for flow cytometric detection;
ii) If the detection cannot be performed immediately, cells are resuspended in 500µl 0.05-5% formalin, vortex mixed, and stored at 4°C in the dark; and before detection, each of the tubes is added with 2ml PBS solution, centrifuged at 1500-2900 rpm for 5-10 min to discard the supernatant, and added with 400µl pre-cooled PBS-EDTA solution to evenly mix cells for flow cytometric detection.

## Patentansprüche

1. Verfahren zum Nachweis von Lymphozyten in Immunzellen, umfassend die folgenden Schritte:
a) Hinzufügen von mit Immunfluoreszenz-Antikörpern gefärbten Lymphozytenproben zu einer vorgekühlten PBS-EDTA-Lösung und Mischen und Vorbereiten der Zellen für den Nachweis mittels Durchflusszytometrie;
b) Starten und Vorheizen eines Durchflusszytometersystems, Einstellen der Durchflussgeschwindigkeit auf einen niedrigen Geschwindigkeitsbereich von 10-29 µl/min oder einen mittleren Geschwindigkeitsbereich von 30-44 µl/min, dann Zugabe einer PBS-EDTA-Lösung in ein Probenröhrchen, um ein Düsensystem mit einem Flüssigkeitsstrom zu spülen;
c) Hinzufügen der in Schritt a) erhaltenen Probe homogener Zellen zum Probenröhrchen und Testen derselben mit Einstellbedingungen von 3000-20000 Zellen in einem Gate während des Testens und Sammelns von Proben;
die PBS-EDTA-Lösung in Schritt a) oder Schritt b) ist eine Mischlösung, die 0,005-0,05 M PBS und eine Endkonzentration von 2-3 mM EDTA mit einem pH-Wert von 7,2-7,4 enthält;
wobei die Lymphozytenproben für die Durchflusszytometrie-Detektion, die in Schritt a) verwendet werden sollen, nach dem folgenden Verfahren hergestellt werden:
1) Zentrifugation von *in vitro* antikoagulierten Blutproben, um Plasma und Blutzellpräzipitat zu trennen und das Blutzellpräzipitat zu erhalten;
2) Verdünnen des Blutzellpräzipitats mit PBS-Lösung, um eine verdünnte Blutzelllösung zu erhalten;
3) langsames Hinzufügen der verdünnten Blutzelllösung zu einem Zentrifugenröhrchen, das ein gleiches Volumen an Lymphozyten-Trennlösung enthält, und anschließendes Zentrifugieren, um das Plasma zu entfernen und die mittlere Buffy-Coat-Zelllösung zu erhalten;
4) Zugabe der in Schritt 3) erhaltenen Buffy-Coat-Zellen in ein Zentrifugenröhrchen, Zugabe von PBS-Lösung unter Rühren, um eine Suspension zu erhalten, Zentrifugation, Entfernung des Überstands und anschließende Zugabe von PBS-Lösung, um die Zellkonzentration auf 1,5 × 10⁶/ml - 5 × 10⁶/ml einzustellen;
5) Hinzufügen der in Schritt 4) erhaltenen Lymphozytenzellen nach Einstellen ihrer Konzentration in ein Durchflussröhrchen; und Hinzufügen von Durchflusszytometrie-Antikörper zum Markieren von Lymphozyten-Subpopulationen, gleichmäßiges Mischen, Stehenlassen bei 2-8 °C und Inkubieren ohne Licht; wobei die Antikörper Anti-CD3-Antikörper, Lin1 [Lineage Cocktail 1], Anti-CD123-Antikörper, Anti-CD11-Antikörper, CD3-FITC, CD8-PerCP und CD4-PerCP umfassen;
6) Hinzufügen von PBS-Lösung, Mischen und Zentrifugieren, um ungebundene Antikörper zu entfernen;
7) Fertigstellung der Proben; wobei
i) wenn eine Detektion mit einem Durchflusszytometer sofort durchgeführt wird, der Überstand verworfen wird und die Zellen gleichmäßig mit einer auf 2-8 °C vorgekühlten PBS-EDTA-Lösung gemischt werden, um eine durchflusszytometrische Detektion durchzuführen;
ii) Wenn die Detektion nicht sofort durchgeführt werden kann, die Zellen durch Zugabe von 0,05-5 % Formalin direkt resuspendiert, gemischt und bei 2-8 °C im Dunkeln stehen gelassen werden; vor der Detektion wird jedem der Röhrchen PBS-Lösung zugegeben, zentrifugiert, um den Überstand zu verwerfen, und PBS-EDTA-Lösung bei 2-8 °C zugegeben, um die Zellen für die Durchflusszytometrie-Detektion gleichmäßig zu mischen;
Die PBS-Lösung ist eine 0,005-0,05 M PBS-Lösung mit einem pH-Wert von 7,2-7,4.

2. Verfahren nach Anspruch 1, **dadurch gekennzeichnet, dass** Schritt a) ferner umfasst: das Volumen der Lymphozytenprobe beträgt 50-200 µl.

3. Verfahren nach Anspruch 1, **dadurch gekennzeichnet, dass** in Schritt b) die Fließgeschwindigkeit auf einen mittleren Geschwindigkeitsbereich von 35-40 µl/min eingestellt wird.

4. Verfahren nach Anspruch 1, **dadurch gekennzeichnet, dass** Schritt b) ferner eine Vorheizzeit von 5-10 Minuten umfasst.

5. Verfahren nach Anspruch 1, **dadurch gekennzeichnet, dass** Schritt c) ferner das Einstellen von Bedingungen von 15000 Zellen im Gate während des Testens und das Sammeln von Proben umfasst.

6. Verfahren nach Anspruch 1, **dadurch gekennzeichnet, dass** Schritt 1) ferner Zentrifugationsbedingungen von 1500-3500 U/min für 5-30 Minuten, vorzugsweise 1500-2900 U/min für 15-20 Minuten, umfasst.

7. Verfahren nach Anspruch 1, **dadurch gekennzeichnet, dass** Schritt 2) ferner das Verdünnen des Blutkörperchenpräzipitats mit PBS-Lösung in einem Volumenverhältnis von 1:0,5 bis 1:2, vorzugsweise 1:1, umfasst.

8. Verfahren nach Anspruch 1, **dadurch gekennzeichnet, dass** Schritt 3) ferner Zentrifugationsbedingungen von 1500-3500 U/min, 10-20 min und 4 °C umfasst; vorzugsweise 1500-2900 U/min, 15-20 min und 4 °C.

9. Verfahren nach Anspruch 1, **dadurch gekennzeichnet, dass** Schritt 4) ferner Zentrifugationsbedingungen von 1500-3500 U/min, 5-20 min und 4 °C umfasst; vorzugsweise Zentrifugationsbedingungen von 1500-2900 U/min, 5-10 min und 4 °C.

10. Verfahren nach Anspruch 1, **dadurch gekennzeichnet, dass** in Schritt 4) PBS-Lösung in das Zentrifugenröhrchen gegeben wird, bis das Volumen 10 ml bis 15 ml beträgt.

11. Verfahren nach Anspruch 1, **dadurch gekennzeichnet, dass** Schritt 6) ferner Zentrifugationsbedingungen von 1500 bis 2900 U/min für 5 bis 10 min umfasst.

12. Verfahren nach Anspruch 1, **dadurch gekennzeichnet, dass** die in Schritt 6) zugegebene Menge an PBS-Lösung 2 ml beträgt.

13. Verfahren nach Anspruch 1, **dadurch gekennzeichnet, dass** die Zentrifugationsbedingungen in Schritt 7) 1500-2900 U/min für 5-10 min betragen.

14. Verfahren zum Nachweis von Lymphozyten in Immunzellen, umfassend die folgenden Schritte:
a) Zugabe von mit Immunfluoreszenz-Antikörpern gefärbten Lymphozytenproben zu einer vorgekühlten PBS-EDTA-Lösung und Mischen und Vorbereiten der Zellen für den Nachweis mittels Durchflusszytometrie;
b) Starten und Vorheizen eines Durchflusszytometersystems, Einstellen der Durchflussgeschwindigkeit auf einen niedrigen Geschwindigkeitsbereich von 10 bis 29 µl/min oder einen mittleren Geschwindigkeitsbereich von 30 bis 44 µl/min, dann Zugabe einer PBS-EDTA-Lösung in ein Probenröhrchen, um ein Düsensystem mit einem Flüssigkeitsstrom zu spülen;
c) Hinzufügen der in Schritt a) erhaltenen Probe homogener Zellen zum Probenröhrchen und Testen derselben mit Einstellbedingungen von 3000-20000 Zellen in einem Gate während des Testens und Sammelns von Proben;
die PBS-EDTA-Lösung in Schritt a) oder Schritt b) ist eine Mischlösung, die 0,005-0,05 M PBS und eine Endkonzentration von 2-3 mM EDTA mit einem pH-Wert von 7,2-7,4 enthält;
wobei die Lymphozytenproben für die Durchflusszytometrie-Detektion in Schritt a) nach dem folgenden Verfahren hergestellt werden:
(I) Isolierung von menschlichen peripheren mononukleären Blutzellen aus peripherem Blut;
(1) Entnahme von 0,2 ml der isolierten ursprünglichen Blutprobe zum Zählen und Aufbewahrung von 1 ml der isolierten ursprünglichen Blutprobe mit gekennzeichneten Informationen bei 4 °C;
(2) Zentrifugieren des Blutes in einem Zentrifugenröhrchen bei 1500-2900 U/min für 15-20 Minuten und Lagerung des oberen Plasmas in einem 2-ml-Kryokonservierungsröhrchen bei -80 °C;
(3) Verdünnen des Zellpräzipitats des restlichen Blutes mit PBS-Lösung im Volumenverhältnis 1:1;
(4) Bereitstellen eines 15-ml-Zentrifugenröhrchen und füllen des Zentrifugenröhrchens mit einer Lymphozyten-Trennflüssigkeit, deren Volumen dem der verdünnten Blutzelllösung entspricht;
(5) Langsame Zugabe der verdünnten Blutzelllösung in das Zentrifugenröhrchen aus Schritt (4) und darauf achten, dass die Trennflüssigkeit gut geschichtet bleibt;
(6) Das Zentrifugenröhrchen in eine Niedriggeschwindigkeitszentrifuge stellen, ausbalancieren und 15-20 Minuten lang bei 1500-2900 U/min zentrifugieren;
(7) Nach der Zentrifugation das obere Plasma langsam mit einer 10-ml-Pipette oder einer 3-ml-Pasteurpipette absaugen und verwerfen;
(8) Die mittleren Buffy-Coat-Zellen durch kreisförmiges Pipettieren in ein neues 15-ml-Zentrifugenröhrchen überführen, PBS-Lösung bis zu einem Volumen von 10 ml hinzufügen, 200 µl der Zellsuspension auf einem Vollblutzellzähler zählen, um anschließend die Zellkonzentration einzustellen, und den Rest der Zellsuspension 5-10 Minuten lang bei 1500-2900 U/min zentrifugieren; und
(9) Zentrifugieren, Verwerfen des Überstands und, entsprechend dem Zählergebnis, Hinzufügen einer geeigneten Menge vorgekühlter PBS-Lösung bei 2-8 °C, um die Zelldichte auf 1,5-2,0 × 10⁶/ml einzustellen;
(II) Vorbereitung der Durchflusszytometrie-Proben
(10) Probenfärbung
In jedes der Durchflussröhrchen werden 100 µl Zellsuspension gegeben, der entsprechende Durchflusszytometrie-Antikörper hinzugefügt, wobei die Antikörper gleichmäßig mit den Zellen vermischt werden, und 10-30 Minuten, vorzugsweise 15-20 Minuten und am bevorzugstten 20 Minuten, im Dunkeln bei 4 °C inkubiert;
(11) Waschen der Probe und Zugabe der Lösung
Nach der Inkubation werden die Zellen in 2 ml PBS-Lösung bei 2-8 °C resuspendiert, 5-10 Minuten lang bei 1500-2900 U/min zentrifugiert und der Überstand nach dem Zentrifugieren verworfen; wobei
i) Wenn die Detektion mit einem Durchflusszytometer sofort erfolgt, werden die Zellen gleichmäßig mit 400 µl vorgekühlter PBS-EDTA-Lösung gemischt und sind dann für die Durchflusszytometer-Detektion bereit;
ii) wenn die Detektion nicht sofort erfolgen kann, die Zellen in 500 µl 0,05-5 % Formalin resuspendiert, vortexgemischt und bei 4 °C im Dunkeln gelagert werden; und vor der Detektion wird in jedes der Röhrchen 2 ml PBS-Lösung gegeben, bei 1500-2900 U/min für 5-10 Minuten zentrifugiert, um den Überstand zu verwerfen, und 400 µl vorgekühlte PBS-EDTA-Lösung hinzugefügt, um die Zellen für die durchflusszytometrische Detektion gleichmäßig zu mischen.

## Revendications

1. Procédé de détection des lymphocytes dans les cellules immunitaires, comprenant les étapes suivantes consistant à :
a) ajouter des échantillons de lymphocytes marqués par un anticorps d'immunofluorescence dans une solution PBS-EDTA pré-refroidie, puis les mélanger et préparer les cellules pour la détection par cytométrie en flux ;
b) mettre en marche et préchauffer un système de cytomètre en flux, ajuster la vitesse d'écoulement à une plage de vitesse faible de 10 à 29 µl/min ou à une plage de vitesse moyenne de 30 à 44 µl/min, puis ajouter la solution PBS-EDTA dans un tube d'échantillon afin de rincer un système de buses à l'aide d'un flux de liquide ;
c) ajouter l'échantillon de cellules homogènes obtenu à l'étape a) au tube d'échantillon et l'analyser dans des conditions de réglage de 3 000 à 20 000 cellules dans une porte pendant l'analyse, et collecter les échantillons ;
la solution PBS-EDTA de l'étape a) ou de l'étape b) étant une solution mélangée contenant 0,005 à 0,05 M de PBS et une concentration finale de 2 à 3 mM d'EDTA, avec un pH de 7,2 à 7,4 ;
les échantillons de lymphocytes destinés à la détection par cytométrie en flux, utilisés à l'étape a), sont préparés selon la méthode suivante consistant à :
1) centrifuger des échantillons de sang anticoagulés *in vitro* pour séparer le plasma et un précipité de cellules sanguines obtenir un précipité de cellules sanguines ;
2) diluer le précipité de cellules sanguines avec une solution de PBS pour obtenir un diluant de cellules sanguines ;
3) ajouter lentement le diluant de cellules sanguines à un tube à centrifuger contenant un volume égal de solution de séparation des lymphocytes, puis le centrifuger pour éliminer le plasma et pour obtenir le diluant de la couche leucocytaire intermédiaire ;
4) ajouter les cellules de la couche leucocytaire obtenues à l'étape 3) dans un tube à centrifuger, ajouter une solution PBS sous agitation pour obtenir une suspension, la centrifuger, éliminer le surnageant, puis ajouter une solution PBS pour ajuster la concentration cellulaire à une valeur de 1,5 × 10⁶/ml à 5 × 10⁶/ml ;
5) ajouter les lymphocytes obtenus à l'étape 4), après ajustement de leur concentration, dans un tube ; et ajouter des anticorps de cytométrie en flux pour marquer les sous-populations lymphocytaires, les mélanger uniformément, les incuber à une température de 2 à 8 °C à l'abri de la lumière ; les anticorps comprenant les anticorps anti-CD3, lin1 [Lineage cocktail 1], les anticorps anti-CD123, les anticorps anti-CD11, CD3 FITC, CD8 PerCP et CD4 PerCP ;
6) ajouter une solution PBS, la mélanger et centrifuger pour éliminer les anticorps non liés ;
7) préparer des échantillons ; sachant que
i) si une détection par cytométrie en flux est effectuée immédiatement, le surnageant est éliminé et les cellules sont mélangées uniformément avec une solution PBS-EDTA pré-refroidie à une température de 2 à 8 °C en vue de la détection par cytométrie en flux ;
ii) si la détection ne peut être effectuée immédiatement, les cellules sont directement remises en suspension par ajout de 0,05 à 5 % de formol, mélangées et incubées à une température de 2 à 8 °C à l'obscurité ; et avant la détection, chacun des tubes est pourvu d'une solution PBS, centrifugé pour éliminer le surnageant, puis pourvu d'une solution PBS-EDTA à une température de 2 à 8 °C pour mélanger uniformément les cellules en vue de la détection par cytométrie en flux ;
et la solution PBS est une solution PBS de 0,005 à 0,05 M avec un pH de 7,2 à 7,4.

2. Procédé selon la revendication 1,
**caractérisé en ce que** l'étape a) comprend en outre que le volume de l'échantillon de lymphocytes est de 50 à 200 µl.

3. Procédé selon la revendication 1,
**caractérisé en ce que**, à l'étape b), la vitesse d'écoulement est ajustée à une plage de vitesse moyenne de 35 à 40 µl/min.

4. Procédé selon la revendication 1,
**caractérisé en ce que** l'étape b) prévoit un temps de préchauffage de 5 à 10 minutes.

5. Procédé selon la revendication 1,
**caractérisé en ce que** l'étape c) consiste à régler les conditions à 15 000 cellules dans la porte pendant l'analyse, et à collecter les échantillons.

6. Procédé selon la revendication 1,
**caractérisé en ce que** l'étape 1) comprend en outre une centrifugation à une vitesse de 1 500 à 3 500 tr/min pendant 5 à 30 min, de préférence à une vitesse de 1 500 à 2 900 tr/min pendant 15 à 20 min.

7. Procédé selon la revendication 1,
**caractérisé en ce que** l'étape 2) consiste en outre à diluer le précipité de cellules sanguines avec une solution PBS dans un rapport volumique de 1:0,5 à 1:2, de préférence de 1:1.

8. Procédé selon la revendication 1,
**caractérisé en ce que** l'étape 3) comprend en outre une centrifugation à une vitesse de 1 500 à 3 500 tr/min pendant 10 à 20 min à 4 °C ; de préférence à une vitesse de 1 500 à 2 900 tr/min pendant 15 à 20 min à 4 °C.

9. Procédé selon la revendication 1,
**caractérisé en ce que** l'étape 4) comprend en outre une centrifugation à une vitesse de 1 500 à 3 500 tr/min pendant 5 à 20 min à 4 °C ; de préférence à une vitesse de 1 500 à 2 900 tr/min pendant 5 à 10 min à 4 °C.

10. Procédé selon la revendication 1,
**caractérisé en ce que**, à l'étape 4), une solution PBS est ajoutée au tube à centrifuger pour obtenir un volume de 10 ml à 15 ml.

11. Procédé selon la revendication 1,
**caractérisé en ce que** l'étape 6) comprend en outre une centrifugation à une vitesse de 1 500 à 2 900 tr/min pendant 5 à 10 min.

12. Procédé selon la revendication 1,
**caractérisé en ce que** la quantité de la solution PBS ajoutée à l'étape 6) est de 2 ml.

13. Procédé selon la revendication 1,
**caractérisé en ce que** la centrifugation à l'étape 7) est effectuée à une vitesse de 1 500 à 2 900 tr/min pendant 5 à 10 min.

14. Procédé de détection des lymphocytes dans les cellules immunitaires, comprenant les étapes suivantes consistant à :
a) ajouter des échantillons de lymphocytes marqués par un anticorps d'immunofluorescence dans une solution PBS-EDTA pré-refroidie, puis les mélanger et préparer les cellules pour la détection par cytométrie en flux ;
b) mettre en marche et préchauffer un système de cytomètre en flux, ajuster la vitesse d'écoulement à une plage de vitesse faible de 10 à 29 µl/min ou à une plage de vitesse moyenne de 30 à 44 µl/min, puis ajouter la solution PBS-EDTA dans un tube d'échantillon afin de rincer un système de buses à l'aide d'un flux de liquide ;
c) ajouter l'échantillon de cellules homogènes obtenu à l'étape a) au tube d'échantillon et l'analyser dans des conditions de réglage de 3 000 à 20 000 cellules dans une porte pendant l'analyse, et collecter les échantillons ;
la solution PBS-EDTA de l'étape a) ou de l'étape b) étant une solution mélangée contenant 0,005 à 0,05 M de PBS et une concentration finale de 2 à 3 mM d'EDTA, avec un pH de 7,2 à 7,4 ;
les échantillons de lymphocytes destinés à la détection par cytométrie en flux, utilisés à l'étape a), sont préparés selon la méthode suivante consistant à :
(I) isoler des cellules mononucléaires du sang périphérique humain ;
(1) prélever 0,2 ml de l'échantillon de sang initial isolé pour le comptage, et conserver 1 ml de l'échantillon de sang initial isolé, présentant des informations de marquage et conservé à 4 °C ;
(2) centrifuger le sang dans un tube à centrifuger à une vitesse de 1 500 à 2 900 tr/min pendant 15 à 20 minutes, et conserver le plasma surnageant dans un tube de cryoconservation de 2 ml à -80 °C ;
(3) diluer le précipité cellulaire du reste du sang avec une solution PBS dans un rapport volumique de 1:1 ;
(4) fournir un tube à centrifuger de 15 ml et remplir le tube à centrifuger avec un volume égal de solution de séparation des lymphocytes et de diluant de cellules sanguines ;
(5) ajouter lentement le diluant de cellules sanguines au tube à centrifuger de l'étape (4), en gardant le liquide de séparation bien stratifié ;
(6) placer le tube à centrifuger dans une centrifugeuse à faible vitesse, l'équilibrer, puis le centrifuger à une vitesse de 1 500 à 2 900 tr/min pendant 15 à 20 minutes ;
(7) après centrifugation, prélever lentement le plasma situé en haut à l'aide d'une pipette de 10 ml ou d'une pipette Pasteur de 3 ml, et l'éliminer ;
(8) prélever les cellules de la couche leucocytaire intermédiaire par pipetage circulaire dans un nouveau tube à centrifuger de 15 ml, ajouter une solution PBS jusqu'à un volume de 10 ml, compter 200 µl de suspension cellulaire sur un compteur de cellules sanguines de sang total pour l'ajustement ultérieur de la concentration cellulaire, et centrifuger le reste de la suspension cellulaire à une vitesse de 1 500 à 2 900 tr/min pendant 5 à 10 min ; et
(9) centrifuger, éliminer le surnageant et, selon le résultat du comptage, ajouter la quantité appropriée de solution PBS pré-refroidie à une température de 2 à 8 °C pour ajuster la densité cellulaire à une valeur de 1,5 à 2,0 × 10⁶/ml ;
(II) préparer des échantillons pour cytométrie en flux ;
(10) marquer les échantillons :
ajouter à chaque tube 100 µl de suspension cellulaire, puis ajouter un anticorps correspondant pour la cytométrie en flux, l'anticorps étant mélangé uniformément aux cellules, et l'incuber à l'obscurité à 4 °C pendant 10 à 30 minutes, de préférence pendant 15 à 20 minutes et de manière particulièrement préférée pendant 20 minutes ;
(11) laver les échantillons et ajouter la solution ;
après incubation, remettre en suspension les cellules dans 2 ml de solution PBS à une température de 2 à 8 °C, puis les centrifuger à une vitesse de 1 500 à 2 900 tr/min pendant 5 à 10 minutes, et éliminer le surnageant après centrifugation ;
sachant que
i) si une détection par cytométrie en flux est effectuée immédiatement, les cellules sont mélangées uniformément à 400 µl de solution PBS-EDTA pré-refroidie, prête pour la détection par cytométrie en flux ;
ii) si la détection ne peut être effectuée immédiatement, les cellules sont remises en suspension dans 500 µl de formol à 0,05 à 5 %, puis mélangées dans un vortex et conservées à 4 °C à l'obscurité ; et avant la détection, 2 ml de solution PBS sont ajoutés à chacun des tubes qui sont centrifugés à une vitesse de 1 500 à 2 900 tr/min pendant 5 à 10 min pour éliminer le surnageant, et 400 µl de solution PBS-EDTA pré-refroidie sont ajoutés pour mélanger uniformément les cellules en vue de la détection par cytométrie en flux.
